# EUROPÄISCHE PATENTSCHRIFT

(11) **EP 1 289 929 B1**
(45) Veröffentlichungstag und Bekanntmachung des Hinweises auf die Patenterteilung: **01.08.2007**
(21) Anmeldenummer: 01947355.2
(22) Anmeldetag: 11.06.2001
(51) Int. Cl.: C07C 215/66, C07D 311/14, C07D 401/06, A61P 13/00

(54) **VERKÜRZTE SYNTHESE VON 3,3-DIARYLPROPYLAMIN-DERIVATEN**
SHORTENED SYNTHESIS OF 3,3-DIARYLPROPYLAMINE DERIVATIVES
SYNTHESE RACCOURCIE DE DERIVES DE 3,3-DIARYLPROPYLAMINES

(30) Priorität: 14.06.2000 DE 10028443
(43) Veröffentlichungstag der Anmeldung: 12.03.2003
(73) Patentinhaber: SCHWARZ PHARMA AG, 40789 Monheim/Rhld. (DE)
(72) Erfinder: MEESE, Claus, 40789 Monheim (DE)
(74) Vertreter: Bausch, Thorsten
(86) Internationale Anmeldenummer: PCT/EP2001/006577
(87) Internationale Veröffentlichungsnummer: WO 2001/096279

(56) Entgegenhaltungen:
- EP-A- 0 957 073
- WO-A-01/35957
- WO-A-98/43942
- US-A- 5 559 269

## Beschreibung

Die Erfindung betrifft ein Verfahren zur Herstellung von Derivaten von 3,3-Diarylpropylaminen der allgemeinen Formel I und sterisch hochreiner, stabiler Zwischenprodukte sowie deren Verwendung zur Herstellung von pharmazeutischen Zusammensetzungen.

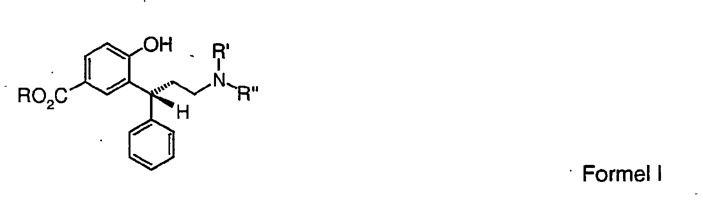

Insbesondere betrifft die Erfindung ein Verfahren zur Herstellung einer Verbindung der Formel II 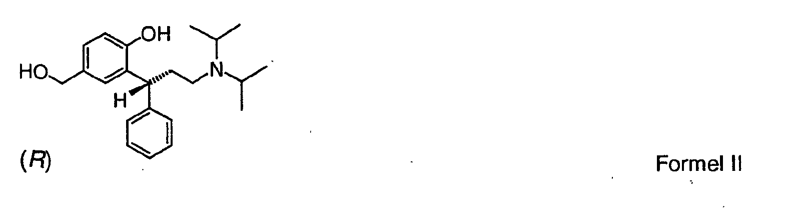 sowie pharmazeutisch wertvolle Zwischenprodukte.

Aus U.S. P 5,559,269 ist ein Verfahren zur Herstellung einer Verbindung der Formel II bekannt. Dieses Verfahren umfaßt 11 Verfahrensschritte bis zum Erhalt dieser Verbindung. Die Gewinnung des R-Enantiomers erfolgt hierbei durch Racemattrennung mit einer chiralen Säure in Verfahrensstufe 5.

Aus PCT/EP99/03212 = WO 99/58478 ist ein Verfahren zur Herstellung einer Verbindung der Formel II bekannt, das 12 Verfahrensstufen umfaßt. Danach erfolgt die Racemattrennung mittels einer chiralen Base in Verfahrensstufe 2.

Beide Verfahren haben die Nachteile, daß sie aufgrund ihrer hohen Zahl an Verfahrensschritten in der Syntheseführung sehr aufwendig sind und zu einer nicht zufriedenstellenden Ausbeute führen.

Aufgabe der vorliegenden Erfindung ist es daher, die vorerwähnten Nachteile zu vermeiden.

Überraschenderweise wurde diese Aufgabe dadurch gelöst, daß ein Verfahren zur Verfügung gestellt wird, das nur noch 6 Verfahrensschritte umfaßt, in dem die erforderliche Racemattrennung bereits in Stufe 1 des Verfahrens erfolgt und das die in den Patentansprüchen genannten Merkmale aufweist.

Zur Herstellung von 3,3-Diarylpropylaminen der oben erwähnten allgemeinen Formel I wird von 4-Hydroxybenzoesäure oder deren Niederalkylestern (PHB-Ester; para-Hydroxybenzoesäureester), vorzugsweise deren Methylester gemäß Formel 1, ausgegangen 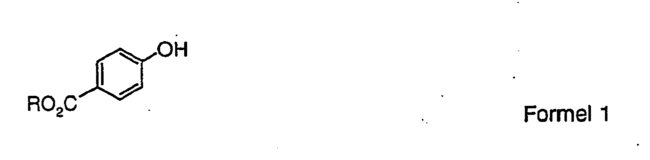 die mit Zimtsäure zu einer Verbindung der allgemeinen Formel 2 umgesetzt werden, 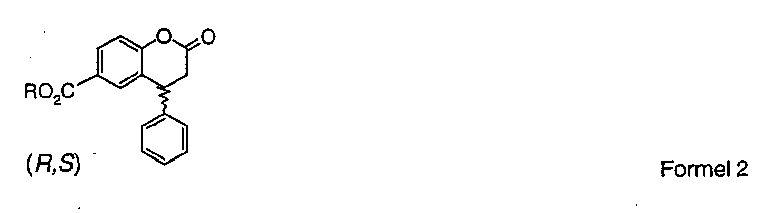 worin
R die Bedeutung von Wasserstoff, geradkettigem oder verzweigtem C₁-C₆-Alkyl, vorzugsweise Methyl oder Isopropyl hat. Durch besondere Reaktionsführung kann verfahrensgemäß, ausgehend von 4-Hydroxybenzoesäureestern, direkt die freie, kristalline Säure gemäß Formel 2a als Reaktionsprodukt erhalten werden.

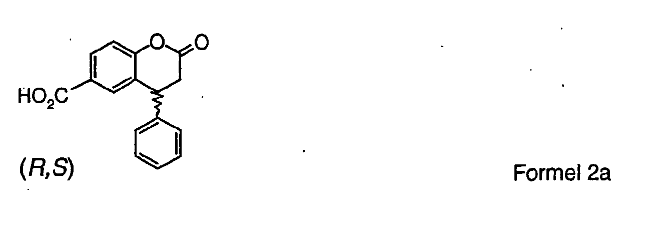

Die Reaktion verläuft in der Wärme und unter Katalyse. Bevorzugtes Lösungsmittel ist Essigsäure. Als Katalysator haben sich Protonensäure wie Schwefelsäure bewährt und günstige Temperaturen liegen zwischen 50°C und 117°C, vorzugsweise 100°C. Die Verbindungen der Formel 2a werden unter den genannten Reaktionsbedingungen als kristalline Feststoffe in etwa 70-78 % Ausbeute und in guter Reinheit (> 90 %) erhalten. Die Reinheit wird durch Umkristallisation z.B. aus 2-Butanon, Essigsäure oder N-Methylpyrrolidin-2-on weiter erhöht.

Mit anorganischen oder organischen Basen werden kristalline Salze erhalten. Chirale organische Basen liefern diastereomere Salze, in denen jeweils ein Enantiomer merklich angereichert ist. Wird das tertiäre, chirale Amin Cinchonidin verwendet, wird in 90 % Reinheit das kristalline Salz gemäß Formel 2b erhalten, in dem zu über 95 % das R-Enantiomer als Säurekomponente überwiegt. Durch weitere Umkristallisation ist eine gesteigerte optische Reinheit von 99 % e.e. erhätlich.

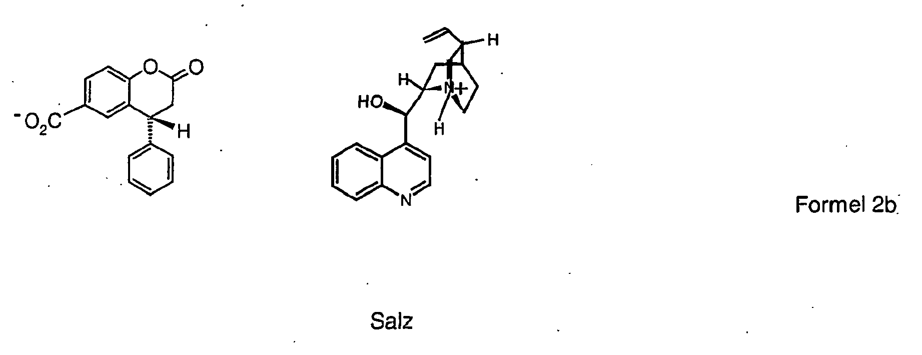

Salze, in denen die Basenkomponente aus anderen chiralen Aminen gebildet wird, führen demgegenüber zu keiner wesentlichen optischen Anreicherung.

Die freie Säure gemäß Verbindung der Formel 3 wird durch Ansäuern von wässrigen Lösungen oder Suspensionen und Extraktion mit einem geeigneten Lösungsmittel isoliert. Erfindungsgemäß wird vorzugsweise Ethylacetat verwendet.

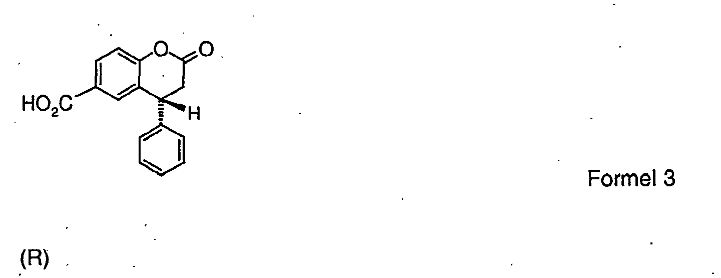

Die reine Verbindung der Formel 3 ist stabil und kristallin. Eine weitere Umkristallisation führt zu hoher chemischer und optischer Reinheit von über 99 %.

Die rechtsdrehende Verbindung der Formel 3 wird nach Aktivierung in Ester der allgemeinen Formel 4, in der R die Bedeutung von geradkettigem oder verzweigtem C₁-C₆-Alkyl, vorzugsweise Methyl oder Isopropyl hat, überführt.

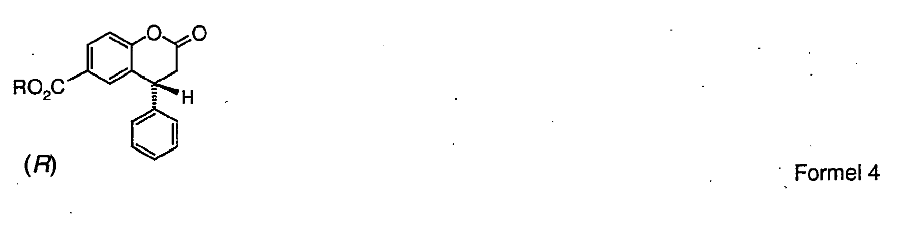

Erfindungsgemäß wird die Umsetzung mit Thionylchlorid oder Oxalylchlorid über die Zwischenstufe eines Säurechlorids durchgeführt, auf die die Esterbildung mit Alkohole des Typs R-OH, in der R die Bedeutung von linearem oder verzweigtkettigem C₁-C₆-Alkyl, bevorzugt Methyl oder Isopropyl hat, in Gegenwart geeigneter Basen folgt.

Die erhaltenen Ester vom Lactontyp fallen als stabile, farblose, kristalline Stoffe an.

Ein weiterer Aspekt der Erfindung ist das gezielte Ausnutzen unterschiedlicher Reaktivität der Carboxylgruppen des Lactonringes und des aromatischen Esters gegenüber Hydridreagenzien.

So erfolgt bei der Einwirkung dieser Reagenzien auf Verbindungen der Formel 4, bevorzugt Diisobutylaluminiumhydrid oder Lithium-tri-tert.butoxyaluminiumhydrid, fast ausschließliche Reduktion des Lactons zu den Lactolen der Formel 5 (beispielhaft: R = Methyl oder Isopropyl)

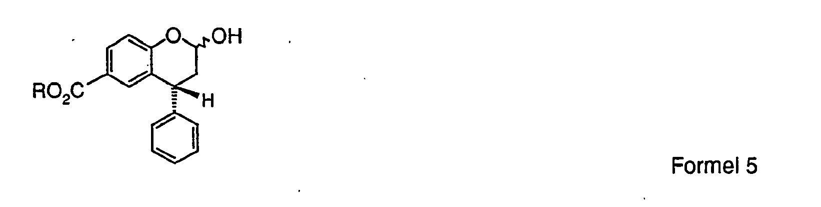

Ein weiterer Aspekt dieser Erfindung ist es, daß unter geeigneten Reaktionsbedingungen aus einer Verbindung der Formel 3 das saure Lactol der Formel 5a entsteht, wenn mit einem Äquivalent Überschuß an Hydridreagenz reduziert wird.

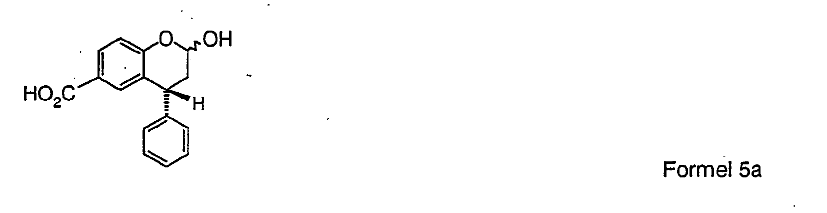

Diese Lactole der Formeln 5 und 5a sind geeignete Substrate für die reduktive Aminierung mit primären, oder sekundären Aminen und führen zu Verbindungen der allgemeine Formel I. In der Formel I haben R' und R" gemeinsam oder verschieden die Bedeutungen von Wasserstoff, geradkettigem oder verzweigtem C₁-C₆-Alkyl, vorzugsweise Methyl oder Isopropyl. R hat darin die bereits vorerwähnte Bedeutung. Bewährt hat sich das System Wasserstoffgas/Edelmetall, bevorzugt Palladium. Auch die Transferhydrierung (Ammoniumformiat/Edelmetall) oder Reduktion mit Hydridreagenzien (z.B. Cyanoborhydrid) können eingesetzt werden.

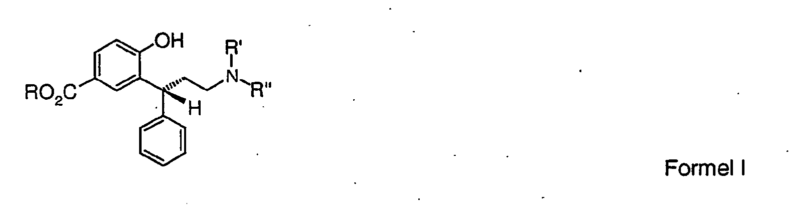

Amine der Formel I lassen sich sowohl als Neutralverbindungen als auch als Salze, vorzugsweise als Hydrochloride, in kristalliner Form erhalten.

Ein weiterer Aspekt der Erfindung ist die Reduktion der Carboxygruppe von Verbindungen der Formel I zu Hydroxybenzylalkoholen der Struktur, wie sie in Formel II wiedergegeben ist. Als Reduktionsmittel eignen sich Diboran, Borhydrid, Aluminiumhydrid, Diisobutylaluminiumhydrid oder bevorzugt Lithiumaluminiumhydrid. Eine Verbindung gemäß

Formel II wird erhalten, wenn R' und R" in Formel I gemeinsam die Bedeutung von jeweils Isopropyl haben.

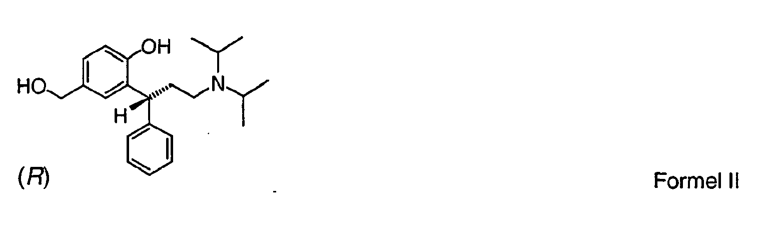

In Analogie zu dem im Dokument PCT/EP99/03212 = WO 99/58478 genannten Herstellprozeß, der auf die hier vorliegende erfindungsgemäße Aufgabenlösung adaptiert wurde, wird das als freie Base rechtsdrehende Enantiomer einer Verbindung der Formel II erhalten. Entsprechend dem anhand der nachfolgenden Ausführungsbeispiele beschriebenen Herstellprozeß für eine Verbindung der Formel II wird das rechtsdrehende Enantiomer erhalten. Dieses besitzt die R-Konfiguration. Der Hydroxybenzylalkohol der Formel II bildet eine Reihe von stabilen, kristallinen Salzen, wobei Hydrochloride, Formiate und Hydrogenfumarate hervorzuheben sind.

Insbesondere die Salze des Hydrogenfumarats eignen sich in hervorragender Weise zur chemisch eleganten Reinigung des Hydroxybenzylalkoholes.

Insbesondere werden nach dem erfindungsgemäßen Verfahren die nachfolgenden Verbindungen hergestellt:
(R,S)-4-Phenyl-2-chromanon-6-carbonsäure
(R)-4-Phenyl-2-chromanon-6-carbonsäure Cinchonidinsalz
(R)-4-Phenyl-2-chromanon-6-carbonsäure
(R,S)-4-Phenyl-2-chromanon-6-carbonylchlorid
(R)-4-Phenyl-2-chromanon-6-carbonylchlorid
(R,S)-2-Oxo-4-phenylchroman-2-carbonsäure-methylester
(R)-2-Oxo-4-phenylchroman-2-carbonsäure-methylester
(R,S)-2-Oxo-4-phenylchroman-2-carbonsäure-isopropylester
(R)-2-Oxo-4-phenylchroman-2-carborisäure-isopropylester
(4R,4S)-2-(R,S)-Hydroxy-4-phenylchroman-6-carbonsäure-methyl ester
(4R)-2-(R,S)-Hydroxy-4-phenylchroman-6-carbonsäure-methylester
(4R,4S)-2-(R,S)-Hydroxy-4-phenylchroman-6-carbonsäure-isopropylester
(4R)-2-(R,S)-Hydroxy-4-phenylchroman-6-carbonsäure-isopropylester
(4R,4S)-2-(R,S)-Hydroxy-4-phenylchroman-6-carbonsäure
(4R)-2-(R,S)-Hydroxy-4-phenylchroman-6-carbonsäure
(R,S)-3-(3-Diisopropylamino-1-phenyl-propyl)-4-hydroxybenzoesäure-methylester - Base und - Hydrochlorid
(R)-(-)-3-(3-Diisopropylamino-1-phenyl-propyl)-4-hydroxybenzoesäure- methylester - Base und - Hydrochlord
(R)-(-)-3-(3-Diisopropylamino-1-phenyl-propyl)-4-hydroxybenzoesäure - Base und - Hydrochlorid
(R,S)-2-(3-Diisopropylamino-1-phonyl-propyl)-4-hydroxymethyl-phenol
(R)-(+)-2-(3-Diisopropylamino-1-phenyl-propyl)-4-hydroxymethyl-phenol-Base, Formiat-und Hydrogenfumaratsalz

Die genannten Verbindungen wurden, wie in der Übersicht des nachfolgend genannten Schemas A dargestellt, gewonnen:

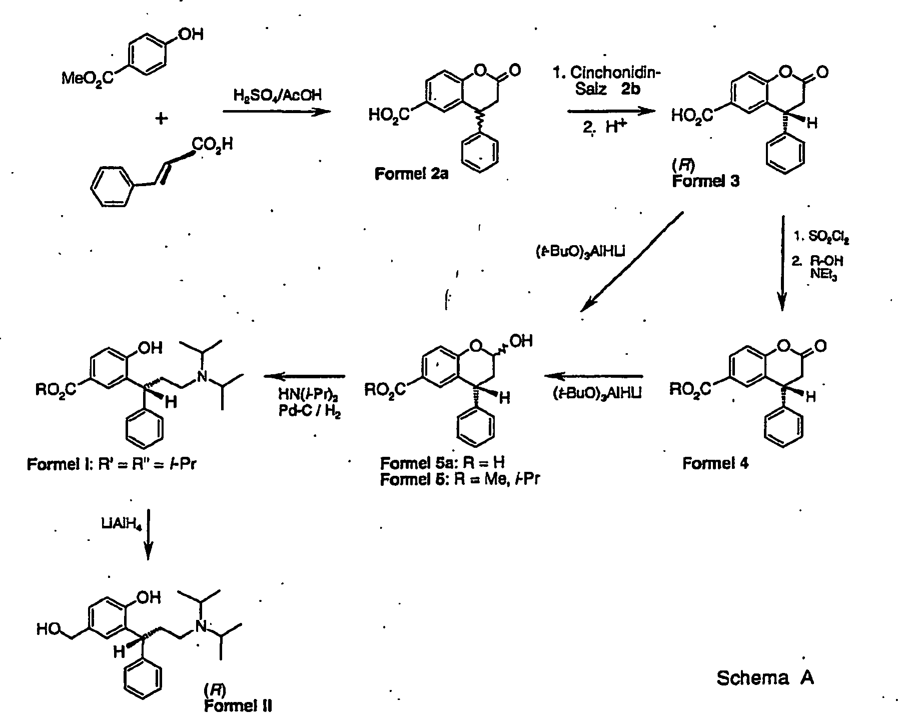

Die Erfindung wird anhand der nachfolgenden Charakterislerungsmethoden und Ausführungsbeispiele näher erläutert:

### I) Charakterisierungsmethoden

Alle beschriebenen Verbindungen wurden vollständig durch ¹H und/oder ¹³C NMR Spektroskopie charakterisiert (Gerät: Bruker DPX 200). Die angeführten Chemischen Verschiebungen für die ¹³C- NMR-Spektren (50 MHz, ppm-Werte) beziehen sich auf die Lösungsmittel CDCl₃ (77.10 ppm), CD₃OD (49.00 ppm) oder Hexadeuterio-dimethylsulphoxid (DMSO-d₆, 39.70 ppm). ¹H-NMR-Daten (200 MHz, ppm) sind auf internes Tetramethylsilan bezogen (0.00 ppm).

### Bestimmung der Enantiomerenreinheit

a) Über HPLC:
   Die Trennungen werden an einer Säule von Daicel (Chiralpak AD, 250x4.6mm) vorgenommen, Eluent ist *n*-Heptan/Ethanol/Trifuoressigsäure (92.5/7.5/0.1, Vol.-%), der Fluß ist 1ml/Min., detektiert wird über UV (250nm). Typische Retentionszeiten, z.B. für die Enantiomeren von (R,S)-1, sind 18.0 bzw. 19.5 Min.
b) Über Kapillarelectrophorese (CE):
   Die Trennungen werden in einem Gerät Typ Beckman-Coulter MDQ an 60cm (ID: 75µm) Kapillaren, bei einem Feld von 500V/cm in einem Puffer von 100m*M*/100n*M* Trispufferl/Borsäure, pH 8.5 in Gegenhart von 3 Gew.-%/Vol. Hydroxypropyl-β-Cyclodextrin-Modifier, durchgeführt. Die Detektion wird über UV bei 200nm vorgenommen. Typische Retentionszeiten der Enantiomere, z.B. die durch alkalische Hydrolyse von (R,S)-1 entstehende Disäure, sind 6.6 bzw. 6.8 Min.
   Die optischen Drehungen wurden bei 589.3 nm und Raumtemperatur ermittelt an einem Perkin Elmer Polarimeter Type 241.
   Die beschriebenen Schmelzpunke (Schmp.) sind unkorrigiert und wurden an einem Mettler FP 1 Instrument aufgenommen, in Einzelfällen auch durch Differentialthermoanalyse (DSC) ermittelt.
   IR Spektren wurden am Perkin-Elmer FTIR Spektrometer Serie 1610 mit einer Auflösung von 4 cm⁻¹ aufgenommen.
   Gaschromatographie-Massenspektroskopie (GC-MS): Die Spektren (m/z-Werte und relative Intensität (%)) wurden am Finnigan TSQ 700 Triple Mass Spectrometer im positiven (P-Cl) oder negativen (N-Cl) Chemical-lonization-Mode aufgenommen mittels Methan oder Ammoniak als Reactant Gas. Hydroxylische Verbindungen wurden analysiert als Trimethylsilylether-Derivative. Verknüpfte Flüssigkeitschromatographie-Massenspectrometrie (LC-MS): Waters Integrety System, Thermabeam Mass Detector (EI, 70 eV), m/z Werte und relative Intensität werden berichtet.
   Elementaranalysen wurden bei der Firma Pascher angefertigt.

### II) Ausführungsbeispiele

1. (R,S)-4-Phenyl-2-chromanon-6-carbonsäure 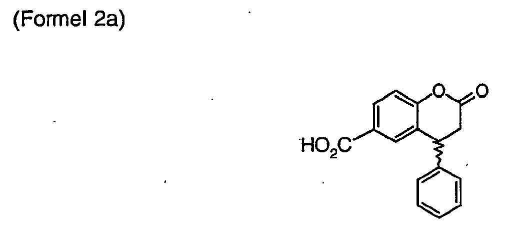
   Eine Mischung aus Zimtsäure (100g, 0.68 Mol), 4-Hydroxybenzoesäuremethylester (108g, 0.71 Mol) und Essigsäure (80ml) wird auf 100 °C erwärmt. Die resultierende klare Lösung wird anschließend unter Rühren mit 80ml 96%-iger Schwefelsäure versetzt. Nach 2 Stunden setzt Kristallbildung ein. Es wird bei gleicher Temperatur weitere 16 Stunden gerührt, auf Raumtemperatur abgekühlt und mit 500ml Wasser verdünnt. Die ausgefallene Kristallmasse wird abfiltriert, mit Diethylether gewaschen und im Vakuum getrocknet.
   Rohausbeute: 142g (78% der Theorie), blaßbeige Kristalle.
   Schmp. 246 °C.
   ¹H-NMR (DMSO-d₆): 3.18 (d, 2H, J = 6.6Hz; CH₂), 4.62 (t, 1 H, J = 6.6Hz, CH), 7.14-7.43 (m, 6H), 7.62 (s, 1 H), 7.90 (d, 1 H, J = 8.6Hz).
   ¹³C-NMR (DMSO-d₆): 35.93 , 39.26, 117.20, 126.81, 127.13, 127.65, 127.70, 129.24, 129.95, 130.25, 140.91, 154.80, 166.66, 167.30
   Strukturbeleg:
   Die Titration mit wässriger 0.1 *N*-NaOH in Dioxan/Wasser gegen Phenolphthalein ergibt ein Äquivalent Carbonsäure/Mol. In der Kapillarelectrophorese zeigt das Electropherogramm einen Hauptpeak (> 90%) für ein einfachgeladenes Anion, nach der alkalischen Hydrolyse ist dieser Peak verschwunden und es tritt ein neuer, gleich intensiver Peak auf mit einer Retentionszeit, die einem Dianion entspricht. Eine methanolische Lösung der Säure wird mit einem Überschuß Triethylamin versetzt und mehrere Tage bei Raumtemperatur belassen. Dünnschichtchromatographisch wird nachgewiesen, daß das Edukt sich zu einem neuen Produkt umgewandelt hat. Das Produkt zeigt in den NMR-Spektren eine Methylesterresonanz.
   Somit handelt es sich bei (*R*,*S*)-1a um das monobasische Säurelacton und nicht um die offenkettige phenolische Disäure.
(R)-4-Phenyl-2-chromanon-6-carbonsäure Cinchonidinsalz 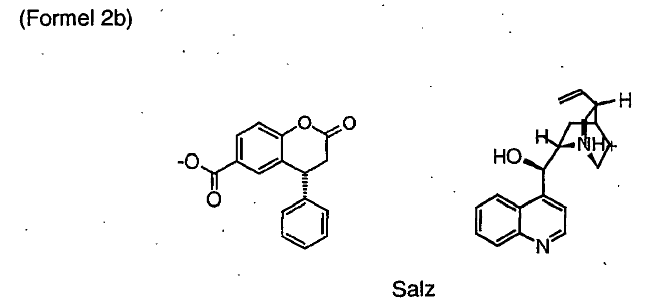
   (R/S)-4-Phenyl-2-chromanon-6-carbönsäure (2.28g, 8.5mmol) und 2.36g (8mmol) Cinchonidin werden in 40ml siedendem 2-Butanon gelöst. Man rührt 18 Stunden bei Raumtemperatur, filtriert die ausgefallenen Kristalle ab und trocknet im Vakuum.
   Ausbeute: 2.13g blaßgelbe Kristalle des Cinchonidin-Salzes der (R)-4-Phenyl-2-chromanon-6-carbonsäure (90% d.Th., e.e. 90%(HPLC)). Umkristallisation aus dem selben Lösungsmittel liefert ein kristallinesSalz mit 99.3% e.e. vom Schmp. 197.5°C.
   ¹³C-NMR (CDCl₃/CD₃OD): 18.17, 24.39, 26.90, 36.86, 37.21, 40.53, 43.32, 54.12, 60.03, 66.23, 116.51, 118.60, 122.70, 124.73, 127.29, 127.41, 128.07, 129.01, 129.31, 129.78, 130.09, 133.02, 137.70, 140.35, 147.20, 149.57, 153.37, 167.64, 172.87.
   [α]_{D}²⁰ = - 38.7 (c = 1.0, MeOH).
3. (R)-4-Phenyl-2-chromanon-6-carbonsäure 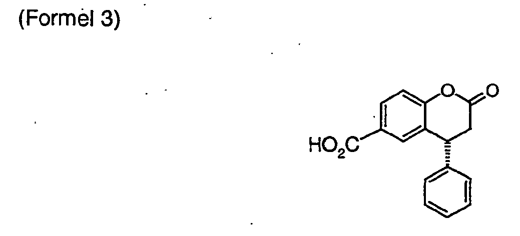
   Eine gerührte Suspension des Salzes 2 in Ethylacetat wird bei Raumtemperatur mit überschüssiger wässriger Salzsäure versetzt. Nach einer Stunde wird die organische Phase abgetrennt, mit Wasser gewaschen und über Natriumsulfat getrocknet. Nach der Filtration wird zur Trockene eingeengt und der kristalline Rückstand aus 2-Butanon/Cyclohexan umkristallisiert. Erhalten werden farblose Kristalle in fast quantitativer Ausbeute (e.e. 99.2%).
   Schmp. 224.9 °C
   ¹³C-NMR (CDCl₃/CD₃OD): 36.43, 40.19, 116.92, 125.54, 126.96, 127.10, 127.57, 128.98, 130.29, 130.59, 139.64, 154.71, 167.28, 167.50.
   [α]_{D}²⁰ = + 45.7 (c = 1.0, MeOH)
4. Carbonylchloride
   a) (R,S)-4-Phenyl-2-chromanon-6-carbonylchlorid 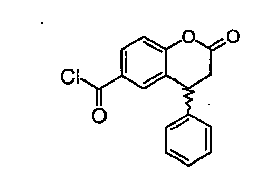
      Eine Mischung von (R/S)-4-Phenyl-2-chromanon-6-carbonsäure (21.5g, 0.08mol) in 80ml Toluol wird mit 4 Tropfen Pyridin und anschließend 17.7ml (0.24mol) Thionylchlorid versetzt. Nach 30 min. Rühren bei Raumtemperatur wird 2 Stunden auf 90-100 °C erhitzt, abgekühlt und am Rotationsverdampfer zur Trockene eingeengt. Der ölige Rückstand wird in Toluol aufgenommen und erneut im Vakuum eingeengt. Zurück bleibt (R/S)-4-Phenyl-2-chromanon-6-carbonylchlorid als blaßgelbes Öl in quantitativer Ausbeute.
      ¹H-NMR (CDCl₃): 3.07 (m, 2H, CH₂), 4.41 (t, 1 H, J = 6.7 Hz,CH), 7.11-7.40 (m, 6H, Aryl-H), 7.59 (d, 1 H, J = 2 Hz, Aryl-H), 8.08 (dd, 1 H, J = 2/6.5 Hz, Aryl-H).
      ¹³C-NMR (CDCl₃): 36.43, 40.51, 118.00, 127.34, 128.23, 129.05, 129.49, 129.56, 132.10, 132.69, 139.12, 165.88, 167.03.
   b) (R)-4-Phenyl-2-chromanon-6-carbonylchlorid
      In gleicher Weise wird (R)-4-Phenyl-2-chromanon-6-carbonsäure zur Titelverbindung umgesetzt, blaßgelbes ÖI.
5. Carbonsäureester
   a) (R,S)-2-Oxo-4-phenylchroman-2-carbonsäure-methylester 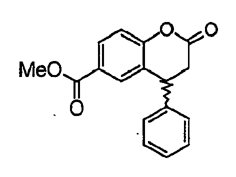
      Eine Lösung von (R/S)-4-Phenyl-2-chromanon-6-carbonsäurechlorid (22.9g, 0.08mol) in absolutem Tetrahydrofuran (100ml) wird bei 0 °C unter Rühren mit 3g (0.094mol) Methanol und 16ml (0.12mol) Triethylamin in 20ml THF versetzt. Nach 18 Stunden Rühren bei Raumtemperatur wird filtriert und das Filtrat zur Trockene eingeengt. Der Rückstand liefert nach der Umkristallisation aus siedendem Diethylether 13.7g (65% d.Th.) (*R*,*S*)-2-Oxo-4-phenylchroman-2-carbonsäuremethylester in Form farbloser Kristalle. Schmp. 97-99 °C.
      ¹³C-NMR (CDCl₃): 36.70, 40.55, 52.19, 117.29, 125.78, 126.67, 127.35, 127.88, 129.29, 130.23, 130.54, 139.79, 155.03, 166.00, 166.60
   b) (R)-2-Oxo-4-phenylchroman-2-carbonsäure-methylester
      Die Umsetzung von (R)-4-Phenyl-2-chromanon-6-carbonsäurechlorid wie für das Racemat beschrieben führt zu kristallinem (R)-2-Oxo-4-phenylchroman-2-carbonsäure-methylester.
   c) (R,S)-2-Oxo-4-phenylchroman-2-carbonsäure-isopropylester 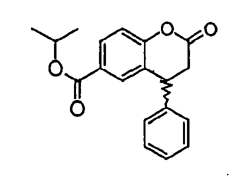
      Der (R,S)-2-Oxo-4-phenylchroman-2-carbonsäure-isopropylester wird in gleicher Weise hergestellt wie für den Methylester beschrieben, blaßbeige Kristalle, Schmp.85.9 °C.
      ¹³C-NMR (CDCl₃): 21.93, 36.88, 40.65, 68.72, 117.25, 125.58, 127.35, 127.53, 127.91, 129.32, 130.30, 130.51, 139.94, 154.95, 165.09, 166.72.
   d) (R)-2-Oxo-4-phenylchroman-2-carbonsäure-isopropylester
      In gleicher Weise wird (R)-4-phenyl-2-chromanon-6-carbonsäurechlorid zu Verbindungen der Formel 4 umgesetzt.
6. 2-Hydroxy-4-phenylchroman-6-carbonsäureester
   a) (4R,4S)-2-(R,S)-Hydroxy-4-phenylchroman-6-carbonsäure-methylester 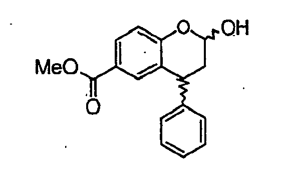
      Eine Lösung von 11.7g (0.042mol) (R,S)-2-Oxo-4-phenylchroman-2-carbonsäuremethylester in 60ml absolutem THF wird bei 0 °C unter Rühren tropfenweise mit einer Lösung von Lithium-tri-tert.-butoxyaluminiumhydrid (11.6g, 0.046mol) in 40ml THF versetzt. Nach zwei Stunden wird der Ansatz auf 100ml Wasser gegossen. Die wässrige Phase wird mehrfach mit Ethylacetat extrahiert, die vereinigten organischen Phasen werden mit Wasser gewaschen, über Natriumsulfat getrocknet und im Vakuum zur Trockene eingeengt. Zurück bleiben 11.1 g (95% d. Th.) (4R,4S)-2-(R,S)-Hydroxy-4-phenylchroman-6-carbonsäure-methylester als farbloses Öl.
      ¹³C-NMR (CDCl₃), Gemisch von Diastereoisomeren (ca. 1:5): 36.15, 36.94, 38.36, 41.05, 51.91, 91.75, 94.81, 117.13, 122.88, 124.95, 125.27, 127.03, 127.24, 128.39, 128.72, 128.88, 129.00, 129.73, 129.93, 131.59, 131.77, 143.13, 143.63, 156.33, 166.98.
   b) (4R)-2-(R,S)-Hydroxy-4-phenylchrorman-6-carbonsäure-methylester
      In gleicher Weise wird (R)-4-Phenyl-2-chromanon-6-carbonsäuremethylester zur Titelverbindung umgesetzt.
   c) (4R,4S)-2-(R,S)-Hydroxy-4-phenylchroman-6-carbonsäure-isopropylester
      In gleicher Weise wird (R,S)-4-Phenyl-2-chromanon-6-carbonsäure-isopropylester zur Titelverbindung umgesetzt.
   d) (4R)-2-(R,S)-Hydroxy-4-phenylchroman-6-carbonsäure-isopropylester
      In gleicher Weise wird (R)-4-phenyl-2-chromanon-6-carbonsäure-isopropylester zur Titelverbindung umgesetzt.
7. 2-Hydroxy-4-phenylchroman-6-carbonsäuren
   a) (4R,4S)-2-(R,S)-Hydroxy-4-phenylchroman-6-carbonsäure 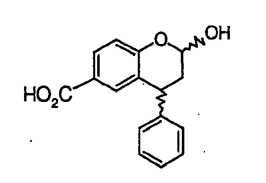
      In gleicher Weise wird (R,S)-4-Phenyl-2-chromanon-6-carbonsäure zur Titelverbindung umgesetzt.
   b) (4R)-2-(R,S)-Hydroxy-4-phenylchroman-6-carbonsäure 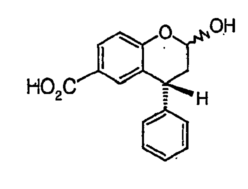
      In gleicher Weise wird (R)-4-Phenyl-2-chromanon-6-carbonsäure zur Titelverbindung umgesetzt.
8. 3-(3-Diisopropylamino-1-phenyl-propyl)-4-hydroxybenzoesäure-methylester
   a) (R,S)-3-(3-Diisopropylamino-1-phenyl-propyl)-4-hydroxybenzoesäure-methylester - Base und Hydrochlorid 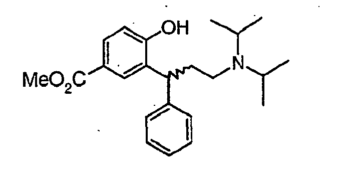
      Eine Lösung von 11.1g (0.039mol) (4R,4S)-2-(R,S)-Hydroxy-4-phenylchroman-6-carbonsäure-methylester und 21 ml (0.15mol) Diisopropylamin in 100ml Methanol wird mit 0.4g Palladium-Kohlekatalysator (10% Pd) versetzt und bei Raumtemperatur und einem Druck von 4 Bar hydriert. Nach dem Spülen der Apparatur mit Stickstoffgas wird die Mischung filtriert, das Filtrat zur Trockene eingeengt und in Diethylether aufgenommen. Die klare, farblose Lösung wird auf 5 °C gekühlt und mit einem leichten Strom von trockenem Chlorwasserstoffgas überspült. Die ausgefallenen farblosen Kristalle von (R,S)-3-(3-Diisopropylamino-1-phenyl-propyl)-4-hydroxybenzoesäure-methylester Hydrochlorid werden abfiltriert und im Vakuum getrocknet (10.9g, 69% d. Th.)
      Schmp. 116.4 °C
      ¹³C-NMR (DMSO-d₆): 16.51, 18.11, 18.78, 31.53, 41.30, 45.81, 51.83, 54.14, 115.60, 120.43, 126.62, 128.01, 128.61, 129.25 129.53,130.01,143.04,159.77,166.30.
      Die freie Base wird durch Verrühren einer Ethylacetatsuspension des Hydrochlorides mit überschüssiger wässriger Natriumcarbonatlösung erhalten. Die organische Phase wird mit Wasser gewaschen, über Natriumsulfat getrocknet, filtriert und zur Trockene eingeengt (Ausbeute quantitativ).
   b) (R)-(-)-3-(3-Diisopropylamino-1-phenyl-propyl)-4-hydroxybenzoesäuremethylester 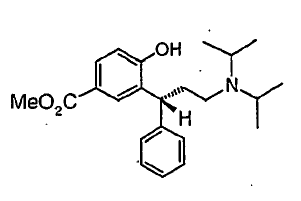
      In gleicher Weise wie für das Racemat beschrieben wird aus (4R)-2-(R,S)-Hydroxy-4-phenylchroman-6-carbonsäure-methylester der optisch aktive (R)-(-)-3-(3-Diisopropylamino-1-phenyl-propyl)-4-hydroxybenzoesäure-methylester in 99.6% Reinheit (HPLC) hergestellt.
      Schmp.: 143.7 °C (DSC: 144.7 °C)
      [α]_{D}²⁰ = - 26.6 (c = 0.93, EtOH)
      ¹³C-NMR (CDCl₃): 18.74, 19.62, 33.12, 39.68, 42.36, 48.64, 51.42, 117.99, 120.32, 126.23, 128.30, 128.85, 129.39, 130.26, 132.21, 144.06, 162.43, 167.35.
      MS (EI, 70 eV): 369 (M⁺, 3%), 354 (13%), 265 (3%), 237 (5%), 209 (5%), 181 (5%), 152 (4%), 126 (8%), 114 (100%).
9. (R)-(-)-3-(3-Diisopropylamino-1-phenyl-propyl)-4-hydroxybenzoesäure 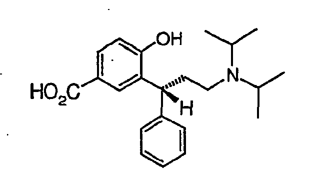 Hergestellt aus (4R)-2-(R,S)-Hydroxy-4-phenylchroman-6-carbonsäure wie oben beschrieben.
   Hydrochlorid: 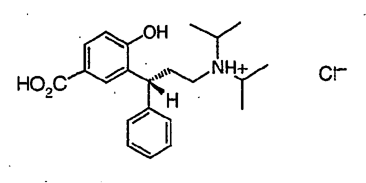
   Hergestellt aus der Base (R)-(-)-3-(3-Diisopropylamino-1-phenyl-propyl)-4-hydroxybenzoesäure und HCl-Gas in Ethylacetat, Umkristallisation aus siedendem Wasser. Schmp.: 260.7 °C
   [α]_{D}²⁰ = - 22.8 (c = 1, MeOH)
   ¹³C-NMR (DMSO-d₆): 16.57, 18.12, 31.68, 41.26, 45.89, 54.19, 115.43, 121.59, 124.99, 126.63, 128.09, 128.65, 129.47, 129.83, 143.17, 159.41, 167.44. Berechnet für C₂₂H₃₀ClNO₃ (Mol.-gew. 391.94): C 67.42%, H 7.72%, Cl 9.05%, N 3.57%, O 12.25%; gefunden: C 65.63%, H 7.68%, Cl 8.54%, N 3.68%, O 12.05%.
10. (R,S)-2-(3-Diisopropylamino-1-phenyl-propyl)-4-hydroxymethyl-phenol 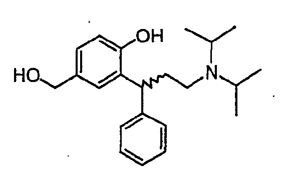
   Eine Lösung von 9.1g (24.6mmol) (R,S)-3-(3-Diisopropylamino-1-phenyl-propyl)-4-hydroxybenzoesäure-methylester in 50ml THF wird bei 0 °C langsam und unter Rühren in eine Suspension von Lithiumaluminiumhydrid (0.94g, 24.7mmol) eingetropft. Nach zweistündigem Rühren wird auf Raumtemperatur erwärmt und weitere 2 Stunden weitergerührt. Der Ansatz wird heruntergekühlt (0 °C) und nacheinander tropfenweise mit 1ml Wasser und 1ml 10%-iger wässriger Natriumhydrogencarbonatlösung versetzt. Nach dem Filtrieren und Waschen des Filterrückstandes mit THF werden die vereinigten organischen Phasen zur Trockene eingeengt. Das verbleibende zähe, blaßgelbe Öl (7.8g) wird in Ethylacetat aufgenommen und die Lösung wird mit 10%-iger wässriger Natriumhydrogencarbonatlösung gewaschen. Nach dem Trocknen (Natriumsulfat), Filtrieren, Einengen wird der Rückstand aus wenig Ethylacetat umkristallisiert, man erhält nach dem Trocknen im Vakuum 5g (59% d. Th.) (R,S)-2-(3-Diisopropylamino-1-phenyl-propyl)-4-hydroxymethyl-phenol in Form blaßbeiger Kristalle.
   Schmp. 112.2 °C
   ¹³C-NMR (CDCl₃): 19.57, 19.95, 33.34, 39.55, 42.13, 48.01, 65.34, 118.50, 126.27, 126.54, 127.50, 128.37, 128.54, 132.61, 132.77, 144.56, 155.46.
11. (R)-(+)-2-(3-Diisopropylamino-1-phenyl-propyl)-4-hydroxymethyl-phenol
   (Formel II) 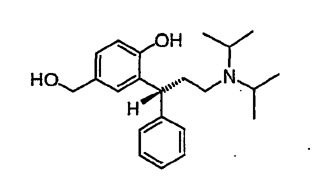
   Hergestellt aus (R)-(-)-3-(3-Diisopropylamino-1-phenyl-propyl)-4-hydroxybenzoesäure-methylester wie oben beschrieben.
   Farblose Kristalle, Schmp. 102.3°C (aus Ethylacetat).
   [α]_{D}²² =+ 21.3 (c = 1.0, EtOH)
12. Formiatsalz 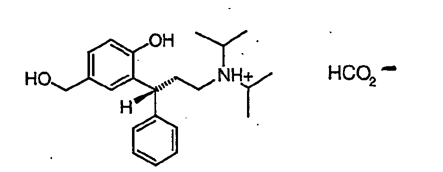
   Zu einer Lösung von (R)-2-(3-Diisopropylamino-1-phenyl-propyl)-4-hydroxymethylphenol (1.02g, 3.0mmol) in 7.5g Acetonitril werden bei Raumtemperatur 138.1 mg (3.Ommol) Ameisensäure zugefügt. Das dabei ausfallende Öl wird durch Erwärmen in Lösung gebracht. Stehenlassen über Nacht führt zur Bildung derber, farbloser Kristalle, die abgesaugt und getrocknet werden (Ausb.: nahezu quantitativ), Reinheit (HPLC): 97.7%.
   Schmp. 151.8 °C
   [α]_{D}²⁰ = - 7.3 (c = 1, Wasser)
   ¹³C-NMR (DMSO-d₆): 19.16, 19.27, 34.21, 41.06, 44.46, 50.78, 63.16, 114.96, 125.72, 126.00, 126.31, 128.08, 128.30, 130.14, 132.96, 144.73, .153.74, 164.98.
13. Hydrogenfumaratsalz 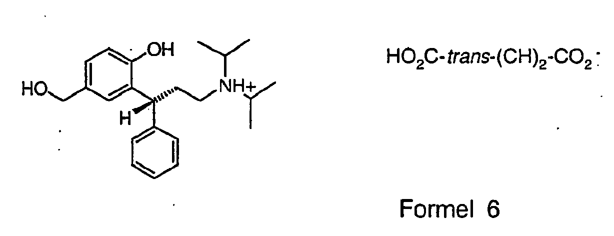
   Zu einer Lösung von (R)-2-(3-Diisopropylamino-1-phenyl-propyl)-4-hydroxymethylphenol (2.94g, 8.61mmol) in 25ml Aceton wird bei Raumtemperatur eine Lösung von 1.0g (8.61mmol) Fumarsäure in 100ml Aceton zugefügt. Das dabei spontan ausfallende farblos kristalline Salz wird noch 30 Minuten bei Raumtemperatur gerührt, abgesaugt und im Vakuum getrocknet.
   Ausbeute: 3.94g (100% d.Th.).
   Schmp. 216.1 °C
   Löslichkeiten bei Raumtemperatur (mg/ml): < 0.2 in Ethylacetat, 2-Butanon, Dichlormethan; > 50 in Methanol; ca. 10 in Wasser.
   ¹³C-NMR (CD₃OD): 17.97, 33.68, 43.19, 47.72, 56.31, 65.05, 116.29, 127.58, 128.04, 128.46, 129.13, 129.53, 130.55, 133.73, 136.29, 144.37, 155.41, 171.38.

## Patentansprüche

1. Verfahren zur Herstellung von 3,3-Diarylpropylaminen der allgemeinen Formel I 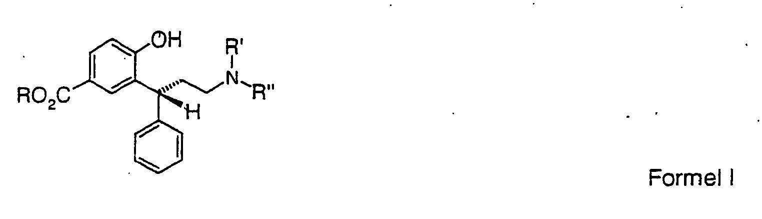 worin
R die Bedeutung von Wasserstoff, geradkettigem oder verzweigtem C₁-C₆-Alkyl, vorzugsweise Methyl oder Isopropyl, R' und R" gleich oder verschieden sein können und die Bedeutung von geradkettigem oder verzweigtem C₁-C₆-Alkyl, vorzugsweise Methyl oder Isopropyl haben, **dadurch gekennzeichnet, daß** eine Verbindung der allgemeinen Formel 1 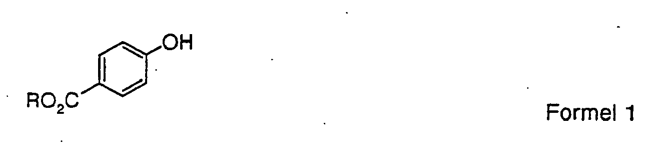 worin R für Wasserstoff, geradkettiges oder verzweigtes C₁-C₆-Alkyl, vorzugsweise Methyl oder Isopropyl steht, mit Zimtsäure zu einer Verbindung der Formel 2a 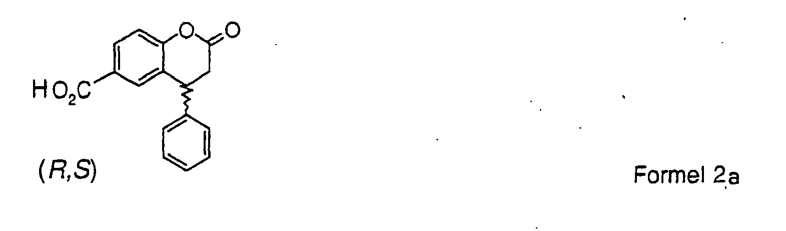 kondensiert wird und anschließend mit einem tertiären chiralen Amin zu einer Verbindung der allgemeinen Formel 2b 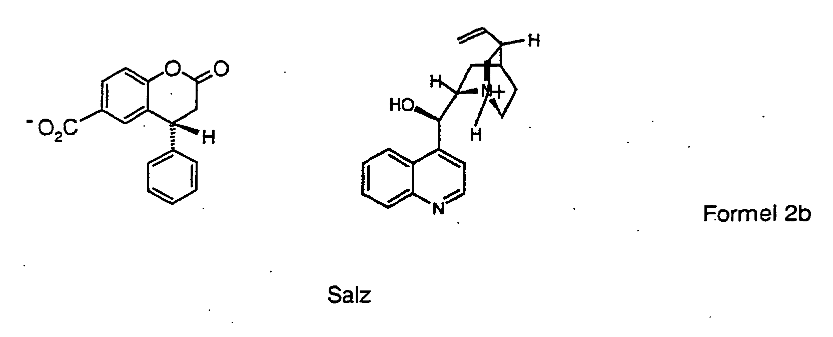 umgesetzt wird und daraufhin durch Ansäuern eine Verbindung der Formel 3 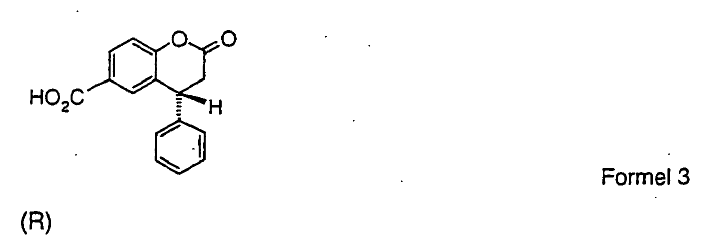 in kristalliner Form isoliert wird, die über die Zwischenstufe eines Säurechlorids unter weiterführender Esterbildung mit Alkoholen des Typs R-OH, worin R für geradkettiges oder verzweigtes C₁-C₆-Alkyl, vorzugsweise Methyl oder Isopropyl steht, umgesetzt wird zu einer Verbindung der Formel 4 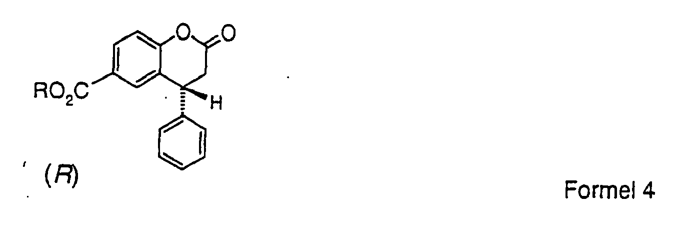 und in einem weiteren Reaktionsschritt mittels Diisobutylaluminiumhydrid oder Lithium-tri-tert.butoxyaluminiumhydrid zu Lactolen der Formel 5 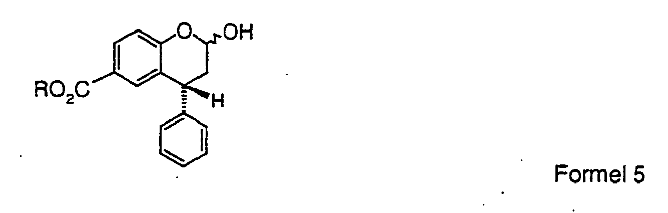 hydriert wird, die schließlich mit primären oder sekundären Aminen zu Verbindungen der allgemeinen Formel I 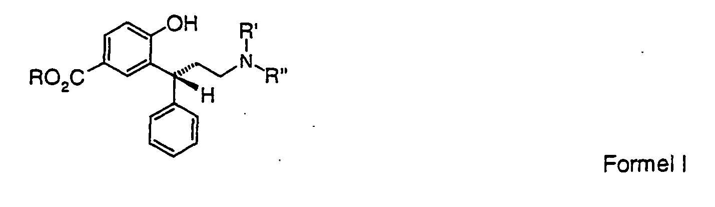 worin
R die Bedeutung von Wasserstoff, geradkettigem oder verzweigtem C₁-C₆-Alkyl, vorzugsweise Methyl oder Isopropyl, R' und R" gleich oder verschieden sein können und die Bedeutung von geradkettigem oder verzweigtem C₁-C₆-Alkyl, vorzugsweise Methyl oder Isopropyl haben,
reduktiv aminiert wird.

2. Verfahren nach Anspruch 1, **dadurch gekennzeichnet, daß** eine Verbindung der Formel 3 mittels Thionylchlorid oder Oxalylchlorid unter Bildung der Zwischenstufe eines Säurechlorids in Gegenwart geeigneter Basen mit Alkoholen des Typs R-OH, worin R die in Anspruch 1 angegebene Bedeutung hat, in eine Verbindung der Formel 4 überführt wird.

3. Verfahren nach Anspruch 1, **dadurch gekennzeichnet, daß** ausgehend von einer Verbindung der Formel 3 unter Verwendung von einem Äquivalent Überschuß an Hydridreagenz direkt zu einer Verbindung der Formel 5a reduziert wird.

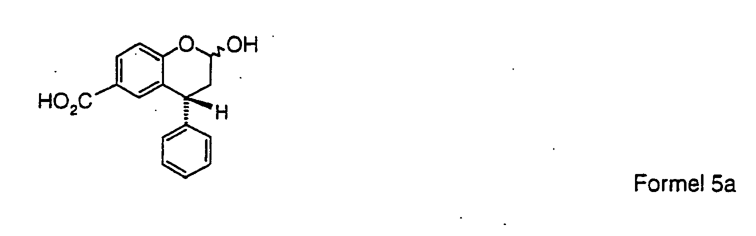

4. Verfahren nach Ansprüchen 1 bis 3, **dadurch gekennzeichnet, daß** eine Verbindung der Formel 1, worin R' und R" gemeinsam die Bedeutung von Isopropyl haben, an deren Carboxygruppe unter Gewinnung einer Verbindung der Formel II 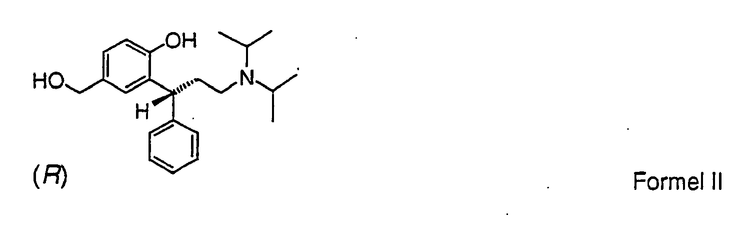 reduziert wird.

5. Verbindung der Formel 2a

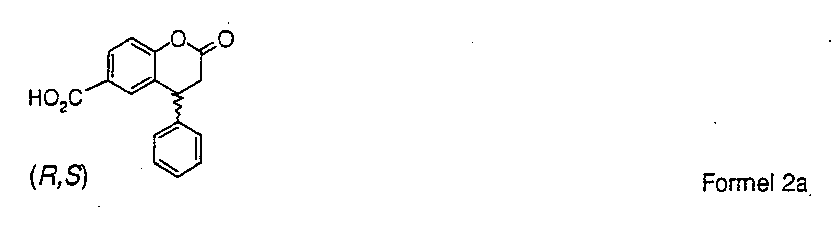

6. Verbindung der Formel 2b

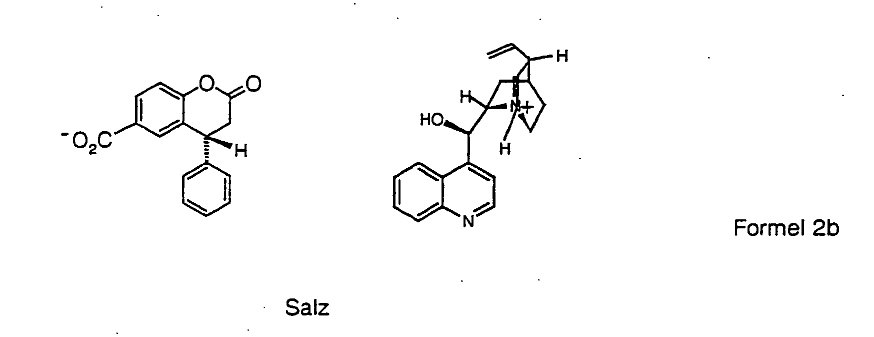

7. Verbindung der Formel 3

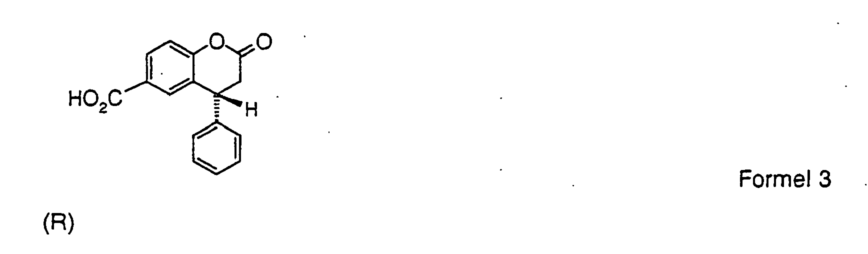

8. Verwendung einer Verbindung nach Ansprüchen 5 bis 7 als Zwischenprodukt zur Herstellung eines rechtsdrehenden Hydroxybenzylalkoholes der Formel II und geeigneter Salze.

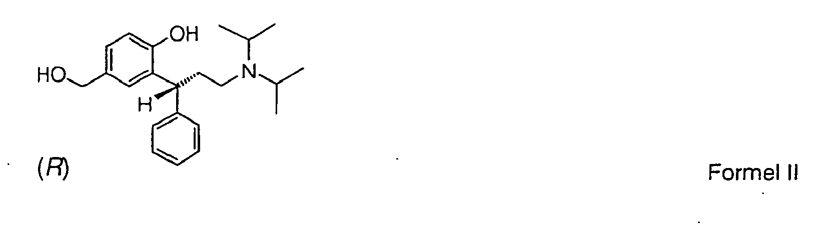

## Claims

1. A process for the production of 3,3-diarylpropylamines of the general formula I 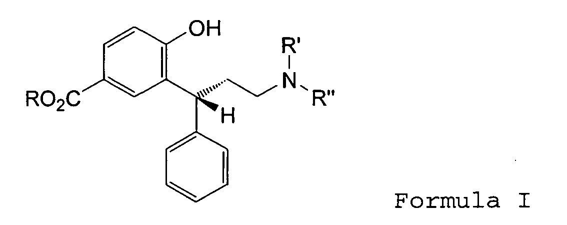 wherein
R represents hydrogen, straight-chain or branched C₁₋₆-alkyl, preferably methyl or isopropyl, R' and R" may be the same or different and represent straight-chain or branched C₁₋₆-alkyl, preferably methyl or isopropyl, **characterized in** condensing a compound of the general formula 1 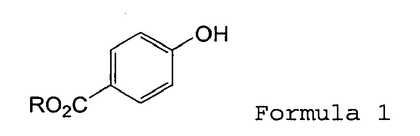 wherein R represents hydrogen, straight-chain or branched C₁₋₆-alkyl, preferably methyl or isopropyl, with cinnamic acid to form a compound of formula 2a 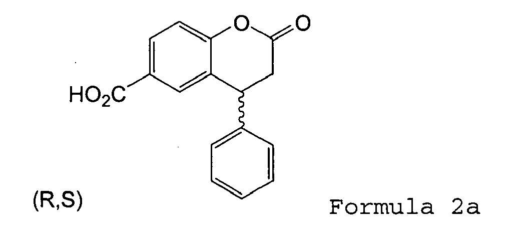 and subsequently reacting it with a tertiary chiral amine to form a compound of the general formula 2b, 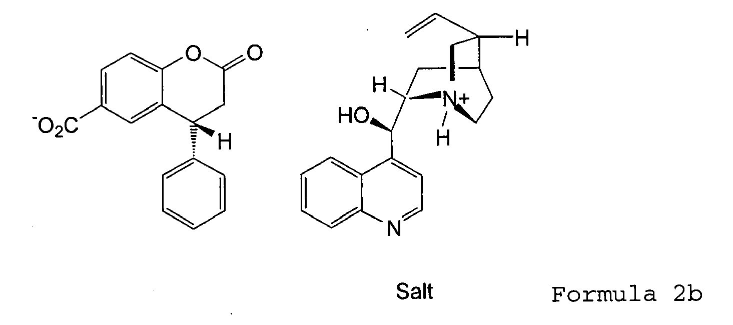 and thereupon isolating a compound of formula 3 in crystalline form by acidification, 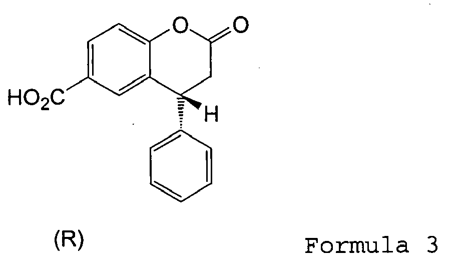 which is converted, via an intermediate acid chloride, with continuing ester formation with alcohols of the formula R-OH, wherein R represents straight-chain or branched C₁₋₆-alkyl, preferably methyl or isopropyl, to form a compound of the general formula 4 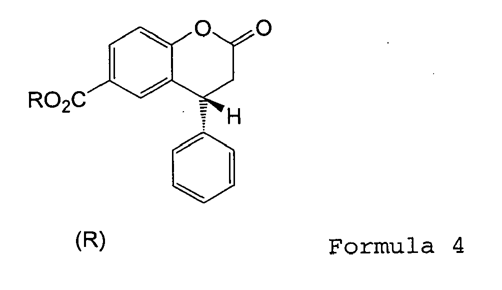 and which, in a further reaction step, is hydrogenated using diisobutylaluminium hydride or lithium tri-tert-butoxyaluminium hydride, to form lactols of formula 5 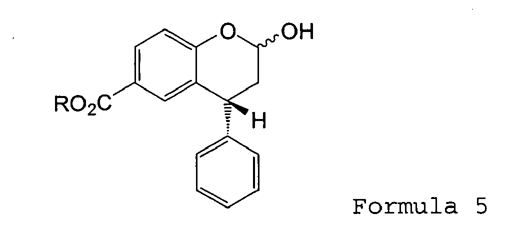 which is finally reductively aminated with primary or secondary amines to form compounds of the general formula I 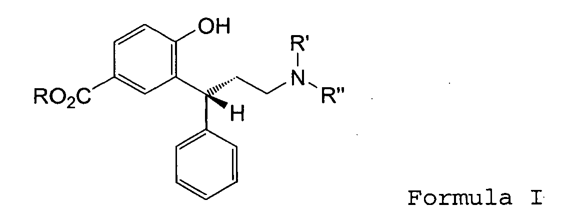 wherein
R represents hydrogen, straight-chain or branched C₁₋₆-alkyl, preferably methyl or isopropyl, R' and R" may be the same or different and represent straight-chain or branched C₁₋₆-alkyl, preferably methyl or isopropyl.

2. The process according to claim 1, **characterized in** converting a compound of formula 3 to a compound of formula 4 using thionyl chloride or oxalyl chloride with the formation of an intermediate acid chloride in the presence of suitable bases with alcohols of the formula R-OH, wherein R is defined as in claim 1.

3. The process according to claim 1, **characterized in** directly reducing the compound of formula 3 to a compound of formula 5a using an equivalent excess of hydride reagent.

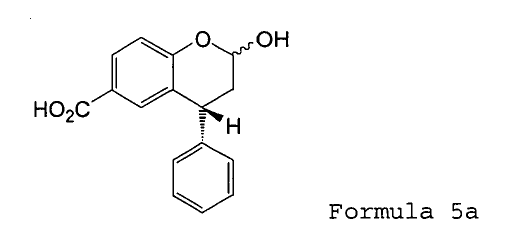

4. The process according to claims 1 to 3, **characterized in** reducing a compound of formula I, wherein R' and R" both represent isopropyl, on its carboxy group to obtain a compound of formula II.

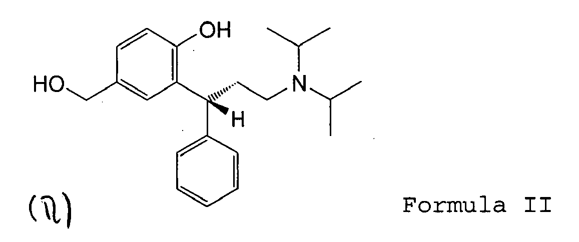

5. A compound of formula 2a:

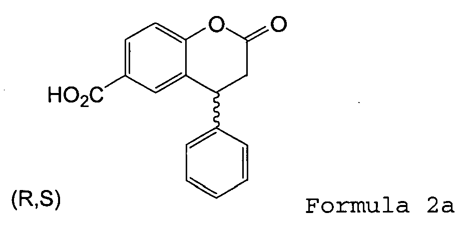

6. A compound of formula 2b:

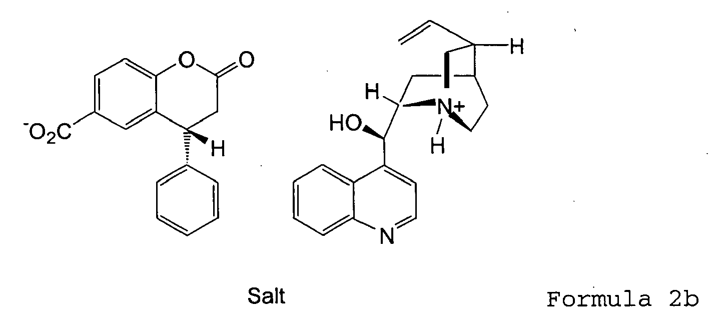

7. A compound of formula 3:

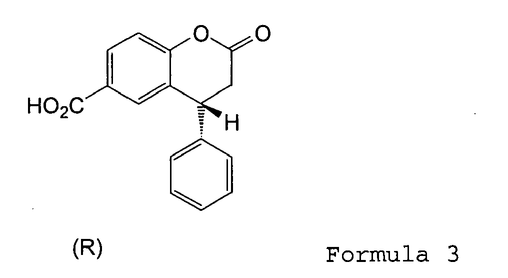

8. Use of a compound according to claims 5 to 7 as an intermediate for the preparation of a dextrorotatory hydroxy benzyl alcohol of formula II and suitable salts.

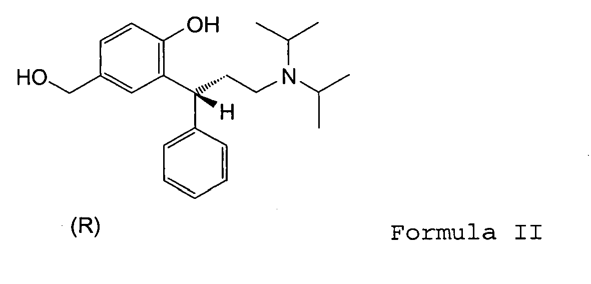

## Revendications

1. Procédé de fabrication de 3,3-diarylpropylamines selon la formule générale I 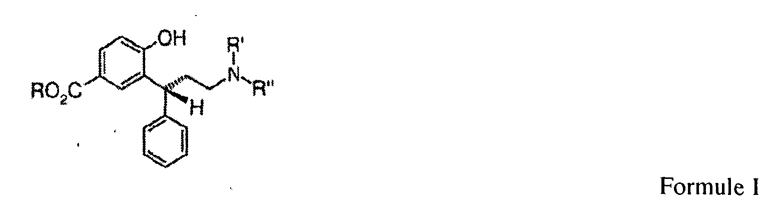 dans laquelle
R désigne l'hydrogène, un alkyle en C₁-C₆ à chaîne droite ou ramifiée, de préférence le méthyle ou l'isopropyle, R' et R" peuvent être identiques ou différents et désignent un alkyle en C₁-C₆ à chaîne droite ou ramifiée, de préférence le méthyle ou l'isopropyle, ***caractérisé en ce qu'un*** composé selon la formule générale 1 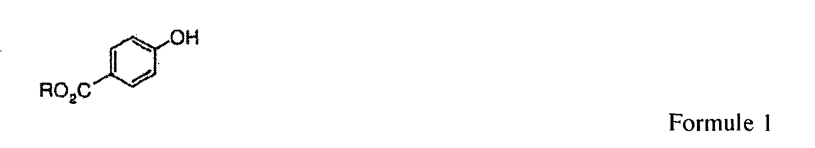 dans laquelle R désigne l'hydrogène, un alkyle en C₁-C₆ à chaîne droite ou ramifiée, de préférence le méthyle ou l'isopropyle, est condensé avec de l'acide cinnamique pour obtenir un composé selon la formule 2a 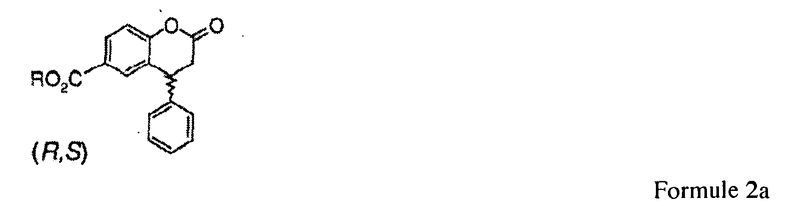 puis transformé à l'aide d'une amine chirale tertiaire en un composé selon la formule générale 2b 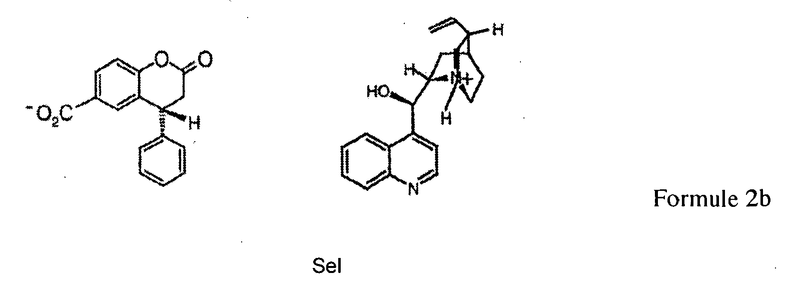 puis un composé selon la formule 3 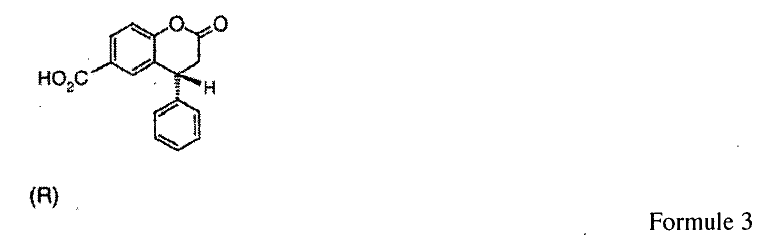 est isolé par acidification sous une forme cristalline, qui est transformée en passant par l'étape intermédiaire d'un chlorure d'acide en poursuivant l'estérification avec des alcools du type R-OH, où R désigne un alkyle en C₁-C₆ à chaîne droite ou ramifiée, de préférence le méthyle ou l'isopropyle, pour donner un composé selon la formule 4 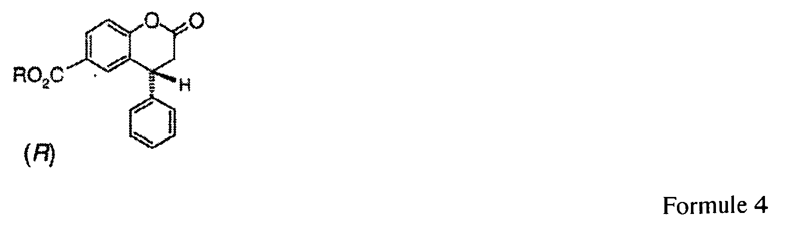 et hydrogéné dans une autre étape de réaction au moyen d'hydrure de diisobutylaluminium ou d'hydrure de lithium-tri-tert.butoxyaluminium pour donner des lactols selon la formule 5 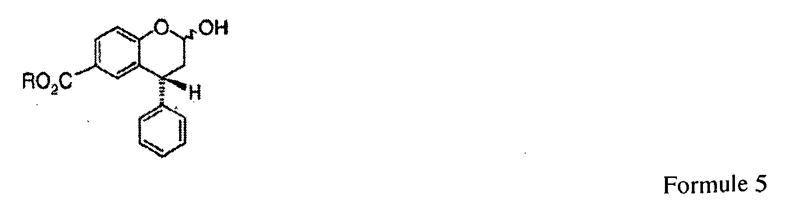 qui sont enfin aminés par réduction avec des amines primaires ou secondaires pour donner des composés selon la formule générale 1 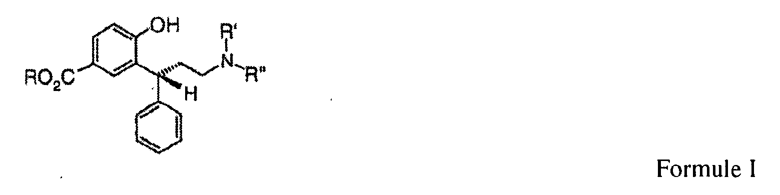 dans laquelle
R désigne l'hydrogène, un alkyle en C₁-C₆ à chaîne droite ou ramifiée, de préférence le méthyle ou l'isopropyle, R' et R" peuvent être identiques ou différents et désignent un alkyle en C₁-C₆ à chaîne droite ou ramifiée, de préférence le méthyle ou l'isopropyle.

2. Procédé selon la revendication 1*, **caractérisé en ce qu'**un* composé selon la formule 3 est transformé en un composé selon la formule 4 au moyen de chlorure de thionyle ou de chlorure d'oxalyle en formant l'étape intermédiaire d'un chlorure d'acide en présence des bases appropriées avec des alcools du type R-OH, où R a la signification indiquée dans la revendication 1.

3. Procédé selon la revendication 1, ***caractérisé en ce qu'**à* partir d'un composé selon la formule 3, en utilisant un excédent équivalent de réactif hydrure, on procède à une réduction donnant directement un composé selon la formule 5a

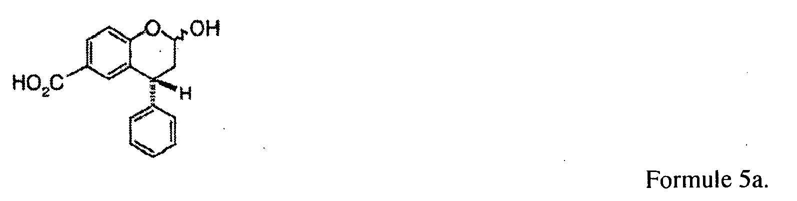

4. Procédé selon les revendications 1 à 3, ***caractérisé en ce qu*'**un composé selon la formule I, dans laquelle R' et R" désignent tous deux l'isopropyle, est réduit sur son groupement carboxy en obtenant un composé selon la formule II

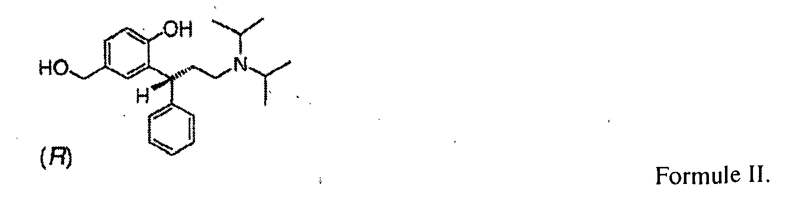

5. Composé selon la formule 2a

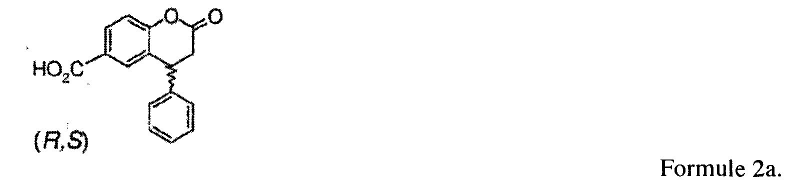

6. Composé selon la formule 2b

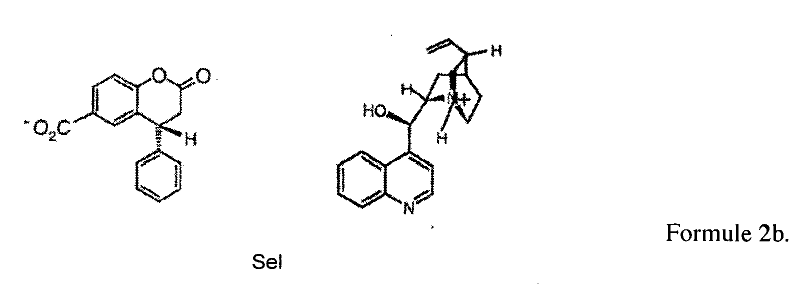

7. Composé selon la formule 3

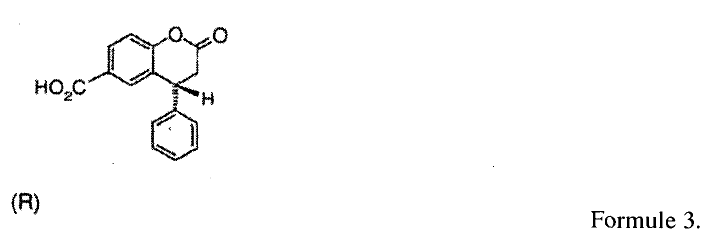

8. Utilisation d'un composé selon les revendications 5 à 7 comme produit intermédiaire pour la fabrication d'un alcool hydroxybenzylique dextrogyre selon la formule II et des sels appropriés
